# EUROPEAN PATENT SPECIFICATION

(11) **EP 1 082 268 B1**
(45) Date of publication and mention of the grant of the patent: **29.12.2004**
(21) Application number: 99920496.9
(22) Date of filing: 13.05.1999
(51) Int. Cl.: C02F 1/32, A61L 2/10, B01J 19/12, A61M 1/36

(54) **ULTRAVIOLET TREATMENT FOR AQUEOUS LIQUIDS**
ULTRAVIOLETTBEHANDLUNG FÜR WÄSSRIGE FLÜSSIGKEITEN
TRAITEMENT AUX ULTRAVIOLETS POUR LIQUIDES AQUEUX

(30) Priority: 13.05.1998 US 76902; 14.12.1998 CA 2255563
(43) Date of publication of application: 14.03.2001
(73) Proprietor: Hallett, Ronald C., Pickering, Ontario L1W 3M5 (CA); Hallett, Douglas J., Acton, Ontario L7J 2L8 (CA)
(72) Inventor: Hallett, Ronald C., Pickering, Ontario L1W 3M5 (CA); Hallett, Douglas J., Acton, Ontario L7J 2L8 (CA)
(74) Representative: VOSSIUS & PARTNER
(86) International application number: PCT/CA1999/000435
(87) International publication number: WO 1999/058453

(56) References cited:
- WO-A-98/05367
- DE-A- 2 753 263
- DE-A- 4 214 994
- DE-A- 4 436 134
- DE-C- 3 806 378
- FR-A- 2 515 655
- FR-A- 2 607 129
- US-A- 3 628 445
- US-A- 5 227 053
- US-A- 5 366 705

## Description

### Field of Invention

The present invention is directed to an ultraviolet (UV) treatment for aqueous liquids such as water or biological fluids.

### Background of Invention

There are many approaches to treating aqueous liquids. The approach taken depends upon a number of factors including the nature of the liquid, the object of the treatment, and the site of treatment, among other factors.

In the case of water to be used for human consumption, the object of treatment might be: to remove certain toxins, such as halogenated hydrocarbons or lead; to reduce the pathogenic content, e.g., render bacteria or viruses less virulent; or to remove components that detract from the taste or smell, but which are otherwise relatively harmless. The site of treatment might be a communal source such as a municipal water treatment plant, or it could be at the point of use, such as in the home.

The present invention involves the use of UV radiation in treatment of aqueous liquids. When the liquid is drinking water, for example, an object is to reduce its pathogenic content. It has been known for quite some time that UV light has bactericidal properties (United States Patent No. 1,193,143, issued August 1, 1916; United States Patent No. 1,200,940, issued October 10, 1916; United States). It is now understood that UV radiation can act to degrade genetic material of a microorganism, i.e., RNA and DNA, to render the microorganism unable to reproduce. This renders the population of microorganisms less virulent and possibly completely harmless to humans.

The use of UV radiation in treating biological fluids is known in a variety of contexts. Exemplary objectives include inactivation of viruses (U.S. Patent No. 5,789,150, issued August 4, 1998) and inhibition of aggregation of blood platelets (U.S. Patent No. 5,591,457, issued January 7, 1997). The treatment might involve a person's own blood (international patent application published as WO/ 98/22164 on May 28, 1998), or the treatment might be in preparation of donated blood or a blood product for administration to another person.

The patent literature describes a large number of apparatuses and methods of UV treatment of aqueous liquids.

One early approach is described in the specification of United States Patent No. 1,193,143, issued August 1,1916 to Henri *et al.* This document describes an apparatus in which a UV lamp is placed outside the liquid and the liquid is caused to flow through a trough. The lamp is provided with a reflector and the sides of the troughs and baffles are made of a reflecting material, in order to utilize the rays emitted from the lamp to their fullest possible extent. In all illustrated arrangements, the lamp is located over the liquid. The liquid is caused to pass and re-pass through the rays in several different ways. In two illustrated embodiments, the liquid is caused to move up and down between baffles. In a third illustrated embodiment, the trough takes the form of a zigzag tube arranged in a horizontal plane. In a fourth illustrated embodiment, the trough is of a spiral form and is arranged so that the liquid in its passage therealong is exposed at all parts to the influence of the UV light.

The specification or United States Patent No. 1,200,940. issued October 10, 1916, also to Henri et *al.,* describes an apparatus in which the W lamp is immersed in the treatment liquid in order to increase efficiency of exposure of the liquid to UV rays. The lamp is protected from contact with the lamp by a quartz window.

The specification of United States Patent No. 1,367,000. issued February 1, 1921 to Pole, describes another apparatus in which the UV lamp is immersed In the treatment liquid. Again, the lamp is shielded from contact with the liquid by a quartz window. In this case, the treatment liquid flows through a narrow channel defined by quartz plates, the channel being located near a UV lamp.

The specification of United States Patent No. 1,473.096, issued November 8, 1923, again to Henri et al. describes an apparatus in which the treatment liquid is passed through one or more compartments located adjacent a UV lamp. Each compartment has a quartz window to permit exposure of the liquid within each compartment to UV light.

The specification of United States Patent No. 2,504,349, issued April 18, 1950 to Prieto, describes a water purification apparatus having a tray which defines a tortuous path which is sloped for the water to travel therealong under the force of gravity. Troughs are defined by the tray to permit the water to travel In a comparatively shallow sheet from the inlet point to the point of discharge. UV lamps are mounted to overlie the troughs. The troughs are formed of a material having high reflecting and low absorption factors. The specification states that the tortuous path which the water takes and the slope of the troughs are such that sufficient time elapses between the delivery of the water to the troughs and its discharge therefrom to enable the UV light from the lamp to be completely effective in disposing of all of the bacteria therein. The angularity or slope of the troughs is such that the water will flow In a stream of substantial uniform depth with a minimum of turbulence throughout its tortuous travel over the tray. There may be a series of parallel (in plan) longitudinal troughs connected in series to each other, or there can be a single trough in the form of a gradually declining spiral. Each lamp is provided with a reflector (semi-circular or parabolic in cross-section) to increase exposure of treatment liquid to UV rays.

The specification of United States Patent No. 4,102,645 describes a sterilization apparatus having a UV lamp located above the liquid being treated, there being a quartz window located between the treatment area and the lamp. An inlet conduit leading into the treatment area is provided with a venturi for introducing air into the liquid. The air is introduced so that an air pocket is maintained above the liquid in the treatment area to prevent direct contact or the liquid with the quartz window and thereby prevent the accumulation of mineral deposits thereon, which deposits would interfere with transmission of UV rays.

The specification of United States Patent No. 3,628,445 (issued to Weber on December 21, 1971) describes treatment of aqueous liquids with UV irradiation in a chamber having a series of walls arranged so that liquid flows through in a zig-zag pattern under the force of gravity. The specification of German Patent Application No. 27 53 263 (published in the name of Hermann Hemscheldt Maschinenfabrik GmbH & Co on May 31, 1979) describes treatment of aqueous liquids with UV irradiation In a chamber having a series of walls arranged so that liquid flows over and under the walls under the force of gravity.

There are UV water purifiers which can be connected in-line to water systems. Examples of such purifiers are described in specifications of United States Patent No. 4,968,437 (issued to Noll *et al*. on November 8, 1990). Canadian Patent Application No. 2,119,543 (published on September 23, 1994 in the names of Kuennen et *al.),* and Canadian Patent Application No. 2,132,929 (published on March 27, 1996 in the name of Szabo).

An example of a system for monitoring the intensity of UV radiation within the treatment chamber of a water purifier is described In the specification of United States Patent No. 4,849,100, which issued to Papendrea on July 18, 1989. The system is suitable for a portable, gravity system in which the UV lamp is housed in a quartz sleeve.

The specification of United States Patent No. 5,039,402, which issued to Himelstein on August 13, 1991, describes a water purifier Incorporated into a household coffee maker.

The specification of United States Patent No. 5,628,895, which issued to Zucholl on May 13, 1997, describes a UV water treatment system in which a UV lamp is located above a container of water.

The use of a laser beam has been suggested by Goudy, Jr., in the specification of United States Patent No. 4,661,264, for disinfection of liquids, typically as part of a larger wastewater treatment facility. Water is passed through a laser beam light produced at a suitable UV wavelength. In one embodiment, the laser source is positioned out of contact with the fluid but with its beam filling the cross-section of the stream of fluid to treat the liquid. A sensor (photocell) is trained at the reflected laser beam and is responsive to the amount of light which is reflected back up toward the surface. The less the light, the greater the turbidity. The photoelectric cell is used to control the oscillator or potentiometer of the laser source and thereby to control the pulse rate of the laser in response to changes in turbidity. Other means for determining turbidity are described. Flow meters are provided which adjust the rate of pulsing of the laser, and therefore the intensity of the ultraviolet light, in relation to changes in flow. This reference also suggests that all interior surfaces of all containers of each embodiment described can advantageously be provided with reflective surfaces to reflect the laser beam and take advantage of the scattering effect which will necessarily result from any suspended particles.

A very recent UV water disinfector is described in the specification of United States Patent No. 5,780,860, which issued to Gadgil *et al.* on July 14, 1998. This approach involves an apparatus having a UV lamp positioned over the water treatment area, and a gravity driven water delivery system is described. The specification mentions that the use of reflectors which redirect UV light toward the feed water offers the advantage of a providing a higher net dosage of UV light to the feed water. Although the approach does not seem to require a thin sheet of water such as that described by Prieto, the specification emphasizes the need for laminar flow of water through the treatment chamber. To this end, a baffle wall is provided at the upstream end of the treatment chamber, the baffle wall having a plurality of spaced perforations to provide for the desired pattern of water flow into the treatment chamber. A reflective wall is provided just downstream of the baffle wall. As characterized in the patent specification, a very low energy UV lamp is all that is required to treat large amounts of water because of the flow design. This reference also teaches that transmittance decreases with increasing turbidity and dissolved salts. It is suggested to monitor turbidity by providing a small visual pattern, such as a square with black and white bars, at the end of an entry feed trough below the water mark. An observer then positions her eyes at the farthest rim of the trough, and observes the lines to determine if they are distinct. If the lines are not distinct, then the liquid is too turbid to be suitable for treatment. Treatment of other fluids is also described by Gadgil *et al.,* for example, elimination of bacterial contaminates in fish culture systems and disinfection of biohazardous liquids such as serum used in producing vaccines to dangerous pathogens.

### Summary of Invention

The present invention is defined by the features of the claims.

In one broad aspect, the present invention is based on the apparently heretofore unrecognized advantages that can ensue from disrupting the flow of a liquid moving at ambient pressure under the force of gravity as it is being treated with UV.

This first aspect of the present invention is thus a process for treating an aqueous liquid as defined in claim 1. The process includes: (1) passing the liquid by force of gravity through a treatment area, the liquid having an upper surface exposed to ambient pressure; (2) disrupting the flow of the tiquid as it passes through the treatment area; and (3) exposing the upper surface of the liquid as the flow is disrupted to UV light. The step of disrupting the flow is carried out so as to direct lower portions of the liquid toward the surface of the liquid to bring such portions into more direct contact with the UV light than would otherwise be the case.

Preferably, the UV light is provided by one or more UV lamps. The range of wavelengths of UV light is understood by the skilled person. UV light having a wavelength of about 254 is known to have germicidal properties.

According to certain embodiments, it is preferred for the liquid to have an average depth of no more than about 3 cm when being treated with UV light. The average depth may also be limited to about 2 cm, about 1 cm, about 0.5 cm or 0.3 cm or less.

Disrupting the flow of liquid involves passing the liquid under the force of gravity down a trough in the treatment area, the trough being shaped to provide physical barriers which purposefully obstruct the even flow of liquid flowing through the trough. The main purpose of the obstructions is to force portions of the liquid resident at the bottom of the trough upwardly toward the surface of the liquid. This brings a greater proportion of the contents of the liquid into close contact with the UV light rays and thus increases the effectiveness of the action of the UV light on the liquid.

Another aspect of the present invention is an apparatus for treating an aqueous liquid such as water with UV radiation as defined in claim 43. The apparatus includes a treatment chamber having an upwardly open trough. The trough defines a flow path for the liquid to flow under the force of gravity under ambient pressure. There is an ultraviolet lamp spaced from the flow path to preclude contact of the lamp with the liquid and located to permit exposure of a top surface of liquid in the trough to radiation emitted from the lamp. The trough has a floor which is shaped to disrupt laminar flow and/or to promote uneven flow of the liquid as it passes through the trough to direct lower portions of the liquid in contact with the floor of the trough toward the upper surface of the liquid. The disruption of the flow should be sufficient to mix the components of the liquid over the span of the flow path through the treatment area of the apparatus. The mixing can be as great that the liquid can be described as turbulent, at least as far this term applies to liquids flowing under the force of gravity.

The present invention has been found to be particularly useful in the area of counter top or portable appliances for treating drinking water within a few hours or just prior to consumption. The illustrated embodiment, described in detail below is one such appliance.

Two sensors can be included as part of the apparatus. The first sensor is located and trained to receive UV light emitted from the lamp. The second sensor is located and trained to receive UV light reflected from reflective surface(s) submerged below the surface of the liquid. The apparatus also includes means for determining the intensity of UV light received by the first sensor relative to the intensity of UV light received by the second sensor so as to determine the effectiveness of the treatment.

The precise way in which effectiveness is determined is achievable in a variety of ways, the preferred ways known to the inventors being described below. The advantage of this arrangement is that for a given appliance, say one for treating tap water to ensure its potability, there is no need for a user to test the water being treated to ensure that the treatment is effective. Generally, a consumer appliance of this type would be equipped with a simple indicator that shows if the treatment is effective. An example of such an indicator is a green light emitting diode (LED) that would be turned on when the treatment is working properly. Thus, in a preferred aspect, the apparatus includes an indicator operably connected to the first sensor and to the second sensor to provide an indication of when the UV light received by the second indicator relative to the UV light received by first indicator is below a predetermined level. So long as the UV light received by the second indicator relative to the UV light received by first indicator is not below this predetermined level, the green LED would remain on. Additionally, another, say red LED, could be included to show that when the UV light received by the second indicator relative to the UV light received by the first indicator has fallen below the predetermined level, the red LED would light up, showing that the water being treated might not be safe to drink, and should therefore be discarded.

The apparatus can also include another indicator operably connected to the first sensor to provide an indication of when the UV light received by the first indicator is below a predetermined level. This indicator, say a red LED, would specifically show that the UV lamp of the apparatus is not functioning at the level needed to be certain that the treatment would be effective. This situation could arise when the machine has just been turned on and the lamp is not yet warmed up to the point where it is emitting sufficient UV light. It could also arise when the lamp is broken or worn down and needs to be replaced.

Additionally, the apparatus can include an indicator operably connected to the first sensor to provide an indication of when the UV light received by the first sensor is above a predetermined level. This could be a green LED.

In a specific embodiment, the first sensor is trained to receive UV light rays emitted directly from the lamp. That is, the first sensor is aimed directly at the lamp. A person skilled in the art could, if need be, arrange the components of the apparatus so that the sensor receives rays indirectly from the lamp, say by use of a mirror. As described in detail in connection with the preferred embodiment, an operational principle of this monitoring aspect of this invention is that the sensors receive UV rays from different parts of the treatment area. The first sensor receives rays from the light source, which rays have not been diminished in intensity by absorption by the liquid being treated. The second sensor is oriented to deliberately receive UV rays from the light source that have passed through the liquid being treated and that have been reflected from reflective surface(s) submerged beneath the liquid. It is by comparing the intensity of these two types of rays received by the two sensors that the effectiveness of the treatment is determined.

The second sensor can be trained to receive light rays that form an angle of between 0° and about 150° with light rays emitted from the lamp. The angle might be between 0° and about 120°, between about 45° and about 120°, or between about 80° and about 100°. In the disclosed embodiment, the angle is about 90°, but it might be possible to improve performance by changing this angle.

The apparatus can be a portable table top appliance, say about the size of a conventional drip coffee maker.

The greater the degree of reflectance from the reflective surfaces, the more effective the treatment. This is because the reflected rays make there way back into the liquid being treated and thus increase the dosage of the W rays being applied to the liquid. This is more the case when the liquid itself is highly translucent. Preferably, the reflective surfaces reflect at least 25% of UV light emitted from the lamp in the absence of liquid; better yet, the reflective surfaces reflect at least 40% of UV light emitted from the lamp in the absence of liquid; better still, the reflective surfaces reflect at least 90% or even 95% or more of UV light emitted from the lamp in the absence of liquid.

Different ways of obtaining increased reflectivity are discussed in connection with preferred embodiments. Many types of surfaces, that might be initially thought to be suitable, are not inert to water or other aqueous liquids that are treatable according to the invention. Additionally, even if a surface that were perfectly reflective to UV light and entirely inert to the liquid being treated were found, the possibility still exists of the surface becoming dirty over time. This would lead to decreased UV reflectivity and the need to clean the surface. In the context of preferred aspects of this invention, this would become evident by the lighting up of a red LED when the intensity of UV light received by the second sensor relative to the intensity of UV light received by the first sensor is to determined to be too low. Alternatively, or additionally, a green LED, lit up to indicate proper operation of the apparatus, would go out under such circumstances.

An appliance is thus preferably arranged so that the reflective surfaces can be readily cleaned. In one example of the invention, the surfaces are part of a removable tray. The tray can be cleaned, if required, or replaced by a new tray.

If the apparatus is a portable counter top appliance, it preferably includes a liquid storage chamber located in an elevated location with respect to the treatment chamber. There is one or more apertures in a wall thereof, the apertures being in communication with the treatment chamber to permit, under the force of gravity, controlled flow of a said liquid from the storage chamber to a said trough of the treatment chamber. By controlled flow, is meant that there is a maximum rate at which it is possible for liquid to exit the storage chamber and enter the treatment chamber. In atable top appliance, only so much liquid can fit into the storage chamber and so it is possible for there to be only so much pressure exerted by the liquid, and this determines the maximum rate at which the liquid can enter the treatment chamber through the fixed hole(s).

The process can include determining the intensity of UV light received by the UV light sensor when the treatment area is empty in order to determine whether the surfaces are sufficiently reflective for the treatment to have the predetermined effectiveness. This acts as a check on the condition of the reflective surfaces.

The process can include determining whether the intensity of the UV light emitted from the light source is sufficient for the treatment to have the predetermined effectiveness. Again, in terms of an apparatus in which the process is being carried out, sufficient UV light from the source can be indicated by an activated green LED, for example.

The process can also include providing an indication of the presence of an unsafe operating condition when the intensity of light received by the UV light sensor when the treatment area is empty is below a predetermined level. This can be indicated by activation of a red LED.

The process can include providing an indication of the presence of an unsafe operating condition when the intensity of the UV light emitted from the light source is below a predetermined level. Again, this can also be indicated by activation of red LED.

The process can include providing an indication of the presence of an unsafe operating condition when the intensity of UV light received by the sensor relative to the UV light emitted from the light source is below a predetermined level. Again, this can be indicated by activation of a red LED.

The liquid treated in the process can be any one of several aqueous liquids. In the case of this aspect of the invention, where reflective surfaces are submerged below the liquid, translucent liquids are preferred to be treated. For example, lake water, or tap water that has been chlorinated.

In a preferred process, the treatment has the predetermined effectiveness based on the intensity of the UV light emitted from the light source and the intensity of the UV light received by the sensor when the UV light received by the sensor is above about 70% the intensity of the UV light emitted from the light source.

In a preferred process, the light source is a mercury lamp.

The process can include determining the intensity of UV light received by the second UV light sensor when the treatment area is empty in order to determine whether the surfaces are sufficiently reflective for the treatment to have the predetermined effectiveness.

The process can include determining whether the intensity of the UV light received by the first UV light sensor is sufficient for the treatment to have the predetermined effectiveness.

The process can include providing an indication of the presence of an unsafe operating condition when the intensity of light received by the second UV light sensor when the treatment area is empty is below a predetermined level.

The process can include providing an indication of the presence of an unsafe operating condition when the intensity of the UV light received by the first UV sensor is below a predetermined level.

The process can include providing an indication of the presence of an unsafe operating condition when the intensity of UV light received by the second sensor relative to the intensity of the UV light received by the first sensor is below a predetermined level.

Other aspects of the invention are described in connection with the preferred embodiments and in the claims.

### Brief Description of the Drawings

Preferred embodiments of the invention are described below, with reference to the appended drawings, wherein:
Figure 1 is a perspective view of a first embodiment of a UV purifier for biological fluids or water of the present invention.
Figure 2 is an illustration of a partial cross-sectional side view of the first embodiment of the UV purifier for biological fluids or water of the present invention as shown in Figure 1.

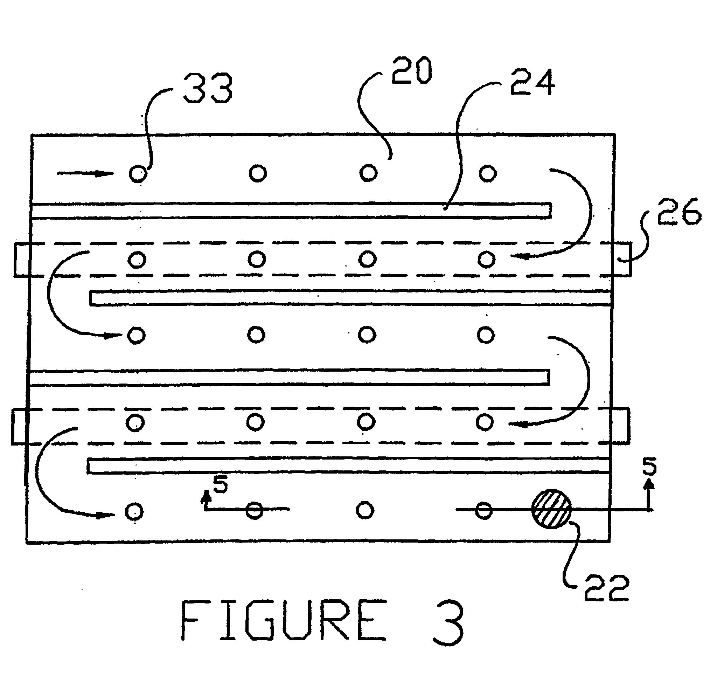

Figure 3 is a plan-type view of a lower tray taken along 3-3 of Figure 2, in which the flow path defined for the liquid is in a zigzag pattern.
Figure 4 is a view, similar to that of Figure 3, of an embodiment of a lower tray having a single wide trough with raised ribs. The embodiment of Figure is not part of the invention as claimed.

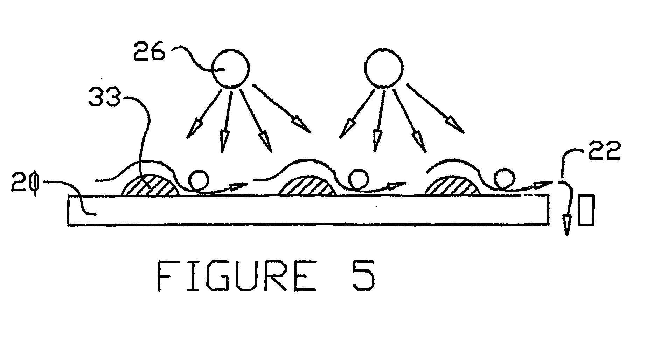

Figure 5, taken along 5-5 of Figure 3 is a representation of liquid flow over upwardly extending ribs located in a trough of the present invention.
Figure 6, is an illustration of a partial cross-section side view of a UV purifier which includes sensors for monitoring the effectiveness of the treatment process.

### Description of Preferred Embodiments the Invention

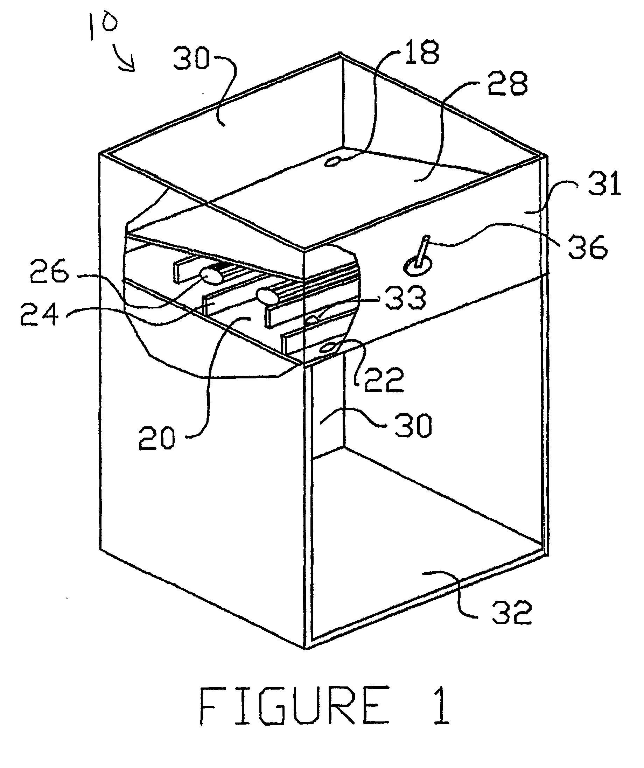

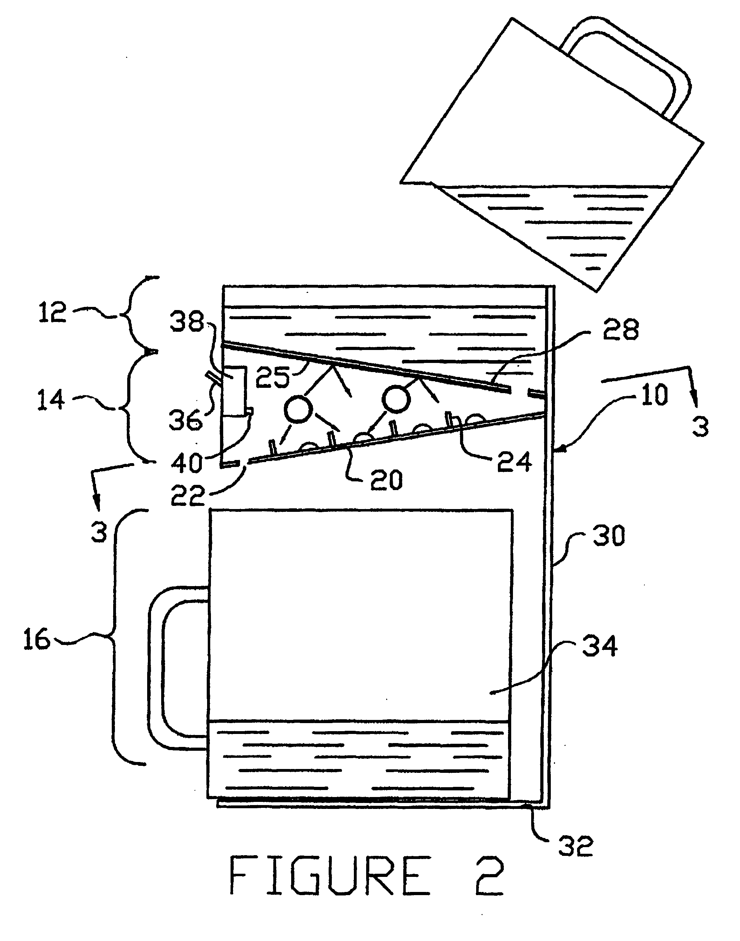

An embodiment of UV apparatus **10** of the present invention is shown in Figures 1 and 2. Apparatus **10** includes upper chamber **12**, middle chamber **14** and lower chamber **16**. The upper chamber has a back wall formed from wall **30**, a front wall formed from wall **31** and a bottom wall or shelf **28**. The top of the upper chamber **12** is open on the top to permit a liquid to be poured into the upper chamber **12**.

Shelf **28** is inclined downwardly toward opening **18** in the shelf. Shelf **28** is not necessarily sloped as shown but the downward incline facilitates liquid from top chamber **12** to flow completely into middle chamber **14**.

The liquid to be treated is poured into upper chamber **12**. Opening **18** in the upper chamber **12** allows the liquid to drain, under the force of gravity, into middle chamber **14** at a controlled rate. Opening **18** is typically provided in a size to provide a liquid flow of up to a rate of about 2 litres per minute. It is possible that there would be more than one opening **18**. To achieve a flow rate of about 2 litres per minute of water it has been observed that a hole having a 6 mm diameter can be used. To maintain the flow rate with higher viscosity fluids, the hole size and/or number of holes are increased or varied as appropriate.

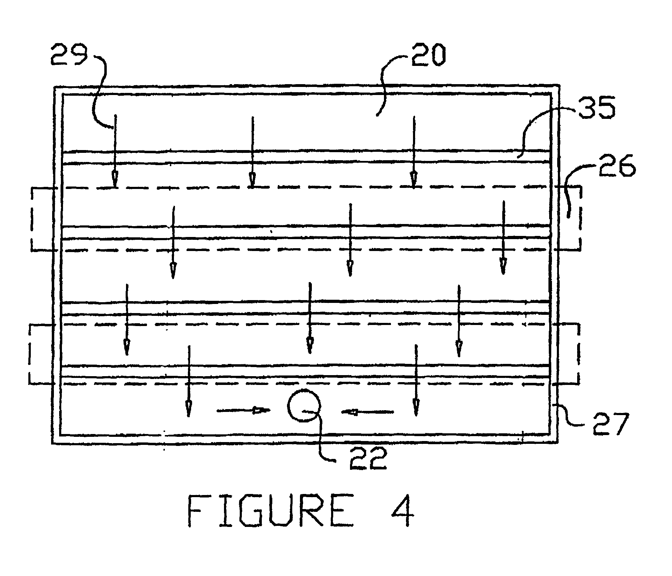

An alternate embodiment (see the tray illustrated in Figure 4) employs multiple openings **18** to obtain a flow rate of 1 to 2 litres per minute. The diameter and/or number of holes can be varied to adjust the flow rate, as desired. Generally, the slower the flow rate and the longer the biological fluid or water is exposed to the UV light the greater likelihood that all bacteria are rendered harmless during treatment.

Middle chamber **14** is defined at the top by the underside of shelf **28**, at the bottom by bottom tray **20**. Middle chamber **14** has a front wall formed from wall **31** and a back wall formed from wall **30**. Bottom tray **20** is slanted downwards to opening **22**. Bottom tray **20** includes walls **24** which together define a trough to channel the liquid flow along a zigzag spaced pattern along tray **20**. The distance "d" (width of the trough) is about 2½ cm. Located in the channels are raised protrusions **33**, which disrupt the even flow of water which comes into contact therewith as it passes along the flow path defined by the trough. The illustrated protrusions are hemispherical and have a height of about 1 cm. Ultraviolet lamps **26** are located in middle chamber **14**. As illustrated, the UV lamps are generally parallel to the flow path of the liquid that travels nearest the lamps. The geometric orientation of the UV lamps, might be altered in an attempt to optimize the effectiveness of UV rays emitted therefrom. It may be found to be advantageous, for example, to include a U-shaped lamp positioned with the legs of the "U" over the second and fourth channels of the trough, for instance.

Preferably, shelf **28** is removable for ready access to middle chamber **14** and UV lamps **26**. This provides for convenient cleaning of tray **20** and replacement of lamps **26**.

In addition, as shown in Figure 2, shelf **28** has a reflective coating **25** on its underside so that reflective coating **25** forms the top surface of middle chamber **14**. Reflective coating **25** reflects the upwardly emitted UV light that strikes it downwardly to increase the amount of UV light striking the top surface of liquid in the treatment area of the apparatus. This increases the exposure of microorganisms sought to be to the UV light. It has been observed that an aluminum reflective coating increases the effective UV dosage.

Apparatus **10** includes power switch **36** for the UV lamps and the ballast **38** for the UV lamps. Power switch **36** is used to turn on and off the power source to UV lamps **26**. The power source can be AC current, DC current and can be provided by any conventional source including batteries or solar panels. Power switch **36** can be situated at any convenient and safe location. Taking into account easy access for the operator and minimal wiring requirements, power switch **36** is shown on the front of middle chamber **14** in Figures 1 and 2.

Lower chamber **16** of the illustrated embodiment is essentially an open space for permitting a hand-held container, e.g., pitcher **34** to be placed for collecting the treated biological fluid or water exiting the opening **22**. Lower chamber **12** includes back wall **30** and bottom **32**. Bottom **32** together with back wall **32** as support provides a base for holding the purifier **10**, when placed on a horizontal surface, in an upright position. It is possible to eliminate lower chamber **16** from the purifier and have upper chamber **12** and middle chamber **14** as a unit which unit would then rest on a container when biological fluid or water was being treated. The biological fluid or water is poured into the top chamber **12** and after being treated in the middle chamber, it exits from the opening **22** in the middle chamber **14**.

If water is being treated, container **34** may be a Brita™ or similar container system capable 5 of removing chemicals and odours and possibly certain metals from the treated water.

The size of the bottom tray **20** must be sufficient to permit the desired flow rate and UV exposure. The bottom tray of the Figure 1 embodiment, which is approximately 22 cm x 15 cm and has four walls **24** to result in five channels, and with four hemispherical spaced apart protrusions **33** (1 cm in height) in each channel of the trough (20 in total) is suitable for treatment of water. Arranging the elements of the apparatus and operating the apparatus at a flow rate of about 1 litre/min with an average depth of water of about 2 cm is thought to be particularly useful.

A number of different configurations of barriers and protrusions can be used to increase the perturbation of even flow of liquid cascading down the trough defined by tray **20**.

The UV lamps required to effectively purify the water from microorganisms may be energized with 110 volts and draw approximately 20 watts during use. However, the power draw is not limited to 20 watts. The power could be drawn from any AC or DC electrical source such as a standard electrical plug, a battery, a solar energy source, etc.

In Figure 2 there is illustrated UV sensor 40. UV sensor **40** is an alarm sensor which indicates when the UV level emanating from the UV lamps **26** is low or nil and as such the UV lamps are not providing a high enough intensity of UV light for effectively rendering the microorganisms in the water harmless.

In the Figure 4 embodiment, a trough is defined between interior walls **27**. The flow path of liquid travelling through the trough of this embodiment is indicated by arrows **29**. The trough is provided with protrusions in the form of upstanding ridges **35** to increase or promote turbulence in liquid flowing over them. In this embodiment, it is not possible for the water to pass through the trough without passing over the protrusions. The ultraviolet lamps are located in middle chamber **14**. Again, the UV lamps can be perpendicular or parallel to the flow of biological fluid or water. Alternatively, the ridges of this embodiment could be smaller and greater in number than that illustrated.

A suitable arrangement has been found to be one in which ridges **35** are about 0.3 cm in height and the water runs over the ridges in a relatively thin film of about 0.2 thickness.

In use, liquid is poured into upper chamber **12**. Liquid flows by gravity into middle chamber **14** through opening(s) **18**. A suitable arrangement is one in which when treating water, if openings **18** are numbered and sized so that the flow of water is a maximum of approximately 2 litres/minute. As the liquid passes through the trough of the middle chamber, it flows over hemispherical protrusions **33** (Figure 1 embodiment of the invention or ridges **35** (Figure 4 embodiment). In each case, even flow of the liquid is disrupted as it travels through the trough and this increases the exposure of the microorganisms in the liquid to the UV light. The reflective coating on the bottom of shelf **28** further increases the UV intensity.

The illustrated apparatus is particularly useful for disinfection of microbiologically contaminated water in lakes/well water or poor municipal waste water systems as found in many developing countries.

Different alarm systems can also be incorporated into the system. For example, an alarm system which that is activated if the UV light is too low or the UV lamp is not turned on when liquid is poured into the top chamber can be incorporated into the apparatus.

Shelf 28 should removable for easy access to the UV lamps for replacing the UV lamps and for cleaning and/or replacing the shelf. Additionally, preferably the bottom tray would also be removable for easy cleaning or for replacing the tray, if necessary.

Preferably all of the materials of construction are resistant to corrosion by the materials with which they potentially come into contact with during the lifetime of the apparatus. Materials for constructions of the upper chamber and trough of the middle chamber of a Figure 1 embodiment to be used with water would thus include suitable plastics, metals and metal alloys. The material should be resistant to leaching. Materials reflective to UV light are also preferred in locations where such reflection will increase the amount of UV light reaching the liquid being treated. This aspect of the invention is described further below.

A spiral shaped flow path similar to that shown in United States Patent No.1,193,143 can be incorporated into the present invention. In such case, of course, the trough is additionally shaped and/or includes protrusions so as to disrupt even flow of the liquid therethrough.

According to the particular embodiments of the present invention disclosed herein, there is no contact between the UV lamp(s) and there is no quartz shield. Thus, there is no need to clean the lamp (or shield) of built up material caused by contact of water. There is the possibility of minor splashing of water onto lamp(s) or quartz shield surfaces, but in the illustrated embodiments, the flow of liquid is sufficiently gentle that there is substantially no splashing of the liquid onto the lamp. An alternative approach to locating the lamp so as to preclude contact with the liquid would be to locate the lamp behind a non-transmissive barrier and reflecting the rays emitted from the lamp to the liquid surface by one or more appropriately situated and suitably reflective surfaces. It may be the case in certain jurisdictions that simply locating the lamp(s) to be out of contact with liquid under normal operating conditions would not be sufficient to meet local safety standards. It may be required that the lamps be shielded by the presence of a physical barrier to take into account deliberate or accidental misuse of the apparatus. In such case, it would still be advantageous to locate the physical barrier (e.g. quartz layer) so as not to come into contact with the treatment liquid under normal conditions as this would reduce cleaning requirements.

The particular embodiments described above have incorporated thereinto what are known In the industry as low pressure UV lamps. These lamps generally have operating temperatures of between about 15 and 40°C. This is not meant to exclude the use of medium pressure lamps as part of the present invention. Medium pressure lamps generally operate at temperatures between about 300 and 900°C. Thus, under most operating conditions, such lamps need cooling. In the case of the present invention, liquid being treated is only in the treatment area for a few seconds, usually about 10 seconds or less, but nonetheless, the apparatus itself may become hotter than desired. Cooling may include ventilating the treatment chamber of the apparatus with chilled or ambient air. The trough can also be cooled, say by the use of an appropriately fitted water jacket, which would generally be located so as not to interfere with light transmission to the treatment liquid.

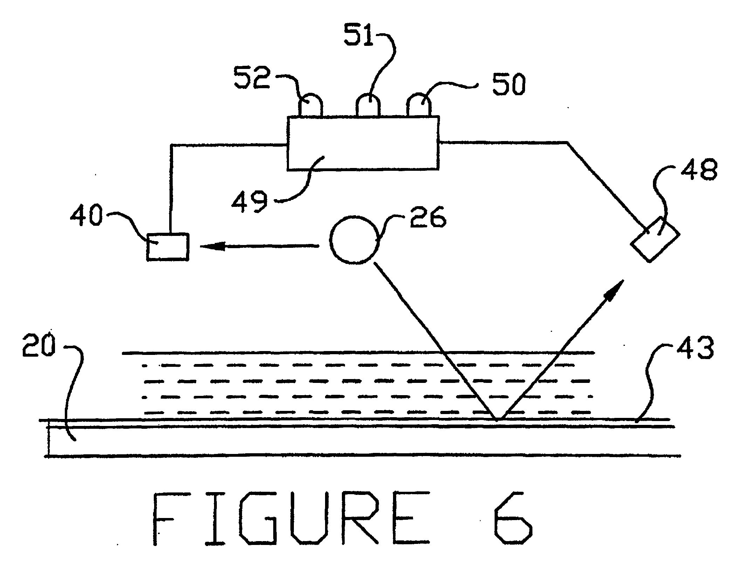

Turning to Figure 6, a UV apparatus including a system to monitor the effectiveness of a treatment being carried out in the apparatus is illustrated. This apparatus, like the other preferred embodiments described herein to illustrate the invention, is for the treatment of a pitcher of water, or the like. The apparatus is thus suitable for treating liquid in a batch process. The apparatus includes a trough, floor **42** of which is illustrated. UV lamp **26** is located above the flow path defined by the trough. The lamp is situated so that the upper surface of liquid flowing through the trough is exposed to UV light being emitted from the lamp. First sensor **40** is spaced from lamp **26** and trained toward the lamp so as to receive UV rays emitted from the lamp without having their intensity reduced, as by absorption, for example. Second sensor **48** is located and trained to receive UV rays that have passed through the treatment liquid and been reflected from the submerged surface **42**. It is generally oriented to receive UV rays that have travelled along the path illustrated. It is to be borne in mind that the indicated path travelled by the UV light is illustrative only and does not take into account diffraction or other effects of the liquid.

The angle 44 of the illustrated embodiment, the angle between the ray incident with the liquid surface and ray striking the sensor, is about 90°. Angle **44** can be varied, by appropriate adjustment of the location and orientation of sensor **48**, from about 0° to possibly as high as about 150°, but an angle intermediate these extremes is more likely to be found to be optimal. Thus the angle is preferably between about 0° and about 120°, more likely between about 45° and about 120°, or between about 80° and about 100°. Sensor **40** is trained to receive UV light rays that are emitted directly from lamp **26**. Optimally, since the difference between the intensities of light received by sensors **40, 48** is important to the operation of the sensors (see below), sensor **40** is situated to receive as little light reflected from the trough as possible.

In the illustrated embodiment, the minimum distance between the centre of the bulb **26** and the floor of the trough is about 3 cm. The distance between the centre of the bulb and first sensor **40** is about 2 cm. The distance between second sensor **48** and the floor of the trough is about 2 cm. The sensors are silicon carbide UV photodiodes. These are obtained from Boston Electronics Corporation of 72 Kent Street, Brookline, MA and available under the model number JEC0.1.

In operation, UV light emitted from the lamp is received directly by sensor **40** and UV light that has passed through the treatment liquid and been reflected by the floor of the trough is received by sensor **48**. The electrical signals are fed to an electronic comparator circuit. During manufacture of the apparatus, the signal from sensor **40** is electronically adjusted with respect to sensor **48**. Thus, while a calibration liquid of a known UV transmissivity is passed through the treatment area, the comparator is adjusted such that, in use, an error signal will be generated if transmissivity is significantly less than that obtained with the calibration liquid. In the illustrated embodiment, a difference in transmissivity is detected when the signal received by sensor **48** (of a calibrated machine) becomes less than that received by sensor **40** by 5 millivolts, which corresponds to roughly a 4% difference in transmissivity.

It will be appreciated that the greater the ability of the floor of the trough to reflect UV light (i.e., to not absorb UV light), the greater will be the effectiveness of a given treatment regimen. This is because the reflected UV rays Will contribute to the effective dosage of UV light bearing upon the liquid being treated. It has been empirically determined by the inventors that stainless steel reflects about 25 percent of UV radiation, a chromed surface about 40 percent and a polished aluminium surface about 90 percent. It would thus appear that of these surfaces, a polished aluminum surface would obtain the best results in the context of a given treatment regimen. One must keep in mind, however, that with time and exposure to elements such as water and its mineral contents, etc., the ability of a surface to reflect UV rays will change, and generally deteriorate.

One particularly promising surface is one obtained by a sputtering process practised by the Commodity Glass of 357 Sutton Place, Santa Rosa, California. In this approach, the tray is of a suitable plastic, say ABS (acrylonitrile-butadiene-styrene), having a thin layer of aluminum bonded to the trough bed surfaces. The aluminum acts as a substrate for a dielectric SiO₂ layer which is applied thereto according to the sputtering process. There may well be other reflective surfaces which have similar or better reflective and durability properties. In a feasibility study, it has been found that such a surface applied to a plastic substrate having an aluminum coating onto which the essentially UV-transparent silicon dioxide coating has been applied, reflects about 95 percent of UV light. The coating is dielectric and is fairly inert to air, water and typical constituents of water to be treated. The coating appears to deteriorate relatively slowly over time.

In any case, a typical preferred operation of the foregoing sensor arrangement is now described in the context of a table top household type appliance that could be used to treat tap water. A device similar to the Figure 1 device is constructed to include the sensor arrangement shown in Figure 6. The arrangement is such that water flows through the device at a maximum rate of about 1.5 litres per minute. Switch **36** is switched on to provide power to the apparatus. Initially, red LED (light emitting diode) **50**, operably connected to sensor **40**, indicates that the power is on, but the intensity of light reaching UV sensor 40 is insufficient for treatment of water. The arrangement provides that when the UV lamp has warmed up to the point that the intensity of its UV output is sufficient for treating water, as determined by UV sensor **40**, the electrical signal of the sensor is high enough to switch off red LED **50** and turn on green LED **51**, indicating to the operator that the device is ready to be used. Third LED **52** is connected to sensors **40** and **48**. This LED, which is red, is activated under the condition when the intensity of light reaching sensor **48** is too low in comparison to that reaching sensor **40**. Thus, if the tray is dirty and insufficiently reflective prior to addition of water to the device, this LED will be activated. Also, for example, if water that is too turbid flows through the treatment area, then LED **52** will light up.

Thus in use, when LED **52** lights up, a user would understand that the treatment may not be yielding potable drinking water. There is a number of situations in which the indicator might be activated: the water may be too opaque to UV light to permit the predetermined amount of light to be transmitted back to sensor **48**; material may have accumulated on the floor of the trough, reducing the amount of UV reflected back to sensor **48**; the reflective surface of the trough may have deteriorated resulting in too high an absorbance of UV light by the floor of the trough. The source of the cause of activation of the indicator would then have to be located and remedied the prior to further use of the device.

A visual inspection of the liquid being treated might indicate whether this is the source of the problem. Alternately, the device could be electrically disabled and disassembled and the tray inspected. If found to be dirty, it could be cleaned with a suitable detergent etc. If the floor of the tray were found by visual inspection to have deteriorated (e.g., corroded or pitted, lost its lustre, etc,) it would generally have to be replaced by a new tray. In a particularly preferred household embodiment, the tray would be removable and replacement trays commercially available. In another embodiment, the input tray is provided with a plurality of inlet ports **18**. It would be possible by plugging one or more of such ports to the slow the rate of flow of liquid through the treatment area. This approach could be taken to obtain a safe operating condition caused by water that were too turbid for treatment (i.e., causes the LED warning light to come on) when all of the ports are open. In the case of household appliances, where not all users would necessarily understand the principle of operation of the apparatus, it would likely be preferred not to provide for such adjustments. That is, for household consumer devices, simplicity of operation would be very important.

In one particular embodiment, an indicator, typically a green LED, is used to indicate when the lamp is properly working. The LED is thus operably connected to sensor 40. In this instance, when the intensity of the UV light reaching sensor 40 is above a predetermined level that is known to be adequate for treating water, the LED would be activated. In some instances, an indicator connected to sensor **40** would be included to indicate when the intensity of the UV light reaching sensor **40** is below a predetermined level. In this case, the indicator might be a red LED or, possibly an audio indicator. The level in this case would be selected so that when the intensity of the UV light being emitted from the lamp falls below a safe operating level, the indicator would be activated, alerting the user to the problem, so that the bulb could be replaced.

In one particular embodiment, an indicator, typically a green LED, is used to indicate when the tray is in proper condition (i.e., sufficiently clean and reflective) for use prior to the addition of water. The LED is thus operably connected to both sensor **40** and sensor **48**. So long as the intensity of the UV light received by sensor **48** is sufficient in comparison to that being emitted by the bulb, the LED would light up. Additionally, another indicator can be included to indicate when the intensity of the UV light received by sensor **48** is insufficient in comparison to that being emitted by the bulb. Here again, the indicator could be a red LED, for example, again indicating a possibly unsafe operating condition. A thus alerted user could thus clean or replace the tray, as necessary.

A particular embodiment of the device would be suitable for use by a person having access to relatively clean but untreated drinking water, such as a lake. Say the water has a high bacteria count of 250 CFU per 100 ml and there is the possibility of crypto sporidium cysts, with an overall UV transmittance of 78% compared to distilled water. The apparatus can include a 20 watt low pressure mercury vapour lamp that produces light with the intensity 2.0mw/cm² as measured by sensor **40**. LED **51**, which indicates that the device is ready for use is set to come on at 1.5 mw/cm². LED **52**, for indicating when UV transmittance through the water being treated is too low, is set to be activated at 70% transmittance, i.e., when the intensity of the light reaching sensor **48** is 70% of that reaching sensor **40**.

A device similar to the Figure 1 device has been shown to produce a UV dose of 90 mw-sec/cm² at 1.5 L/min with a liquid having 75% transmittance compared to distilled water and a lamp output of 1.5 mw/cm². It has also been shown that 38 mw-sec/cm² is sufficient to kill or inactivate all pathogens in water.

To operate this particular embodiment, the user would turn on the device and a red LED would light up and remain on until the lamp is producing sufficiency intense UV light, i.e., until UV output is greater than 1.5 mw/cm² as measured by the sensor trained on the lamp. Once this output is reached, the red LED goes out and the green LED is activated. As water is poured through the device, so long as the green LED remains on, the user can be certain that the water is being properly treated. If UV transmittance falls below 70%, then another red LED will be activated, indicating that something is amiss and the treated water might not be safe to be consumed.

It will thus be understood that a commercial product can be produced according to the invention, which a consumer can use to treat a batch of water and be confident to render any contaminants reasonably foreseen to be contained therein harmless. The required operating parameters of the machine can be set at levels such that the consumer would not be required to check the quality of the water treated with the apparatus.

A person skilled in the art would understand from the foregoing explanation that the two sensors, **40, 48** are generally oriented to receive UV light from the bulb and the UV light reflected from the tray, respectively. The operating parameters of a commercial apparatus are empirically determined and it is the overall operation of the unit that is of importance. For example, a certain amount of "leakage" of UV light between the two sensors is possible while maintaining a safely operating apparatus. Safe operating margins, to take into account possible variations in the quality of water available in different areas can be developed because the operating parameters are empirically determined.

### Examples

In a first trial, an apparatus similar to that illustrated in Figure 1 was used, but in this case, there were no protrusions in the troughs (channels) of the apparatus. Distilled water was spiked with *Bacillus subtilis* spores to give a count of 39,000 CFU's (colony forming units) per ml. The water was poured through the apparatus at a rate of 800 ml/min. The effluent (liquid emerging from the treatment area of the apparatus) had a spore count of 2,200 CFU's per ml. In a comparative run, the same experiment was carried out using the apparatus as illustrated in Figure 1, that is, four evenly spaced semi-circular protrusions (the protrusions were 0.5 cm in height in these examples) were located in the centre of each channel, In this case, the effluent had a spore count of 330 CFU's per ml.

In a second trial, deionized water was spiked with Crypto *sporidium parvum* to a concentration of 10,000 per ml. The water was poured through the apparatus illustrated in Figure 1, again at a rate of about 800 ml/min. In this case. the liquid exiting through port **22** of the apparatus was found to contain no detectable infectious *Crypto sporidium.* The UV dose was calculated to be 100 mWs/cm.

In a third trial, to study the feasibility of treating blood and/or blood products according to the invention, blood serum spiked with *E. coli* bacteria to a count of 2,400,000 CFU's per ml was treated at a rate of 500 ml/min. The blood serum was found to contain 2 *E. coli* CFU's per ml. Similarly, PFU's (plaque forming units) per ml of adenovirus and herpes virus were substantially reduced. The UV transmittance was less than 1% with a UV dose of 120 mWs/cm. No blood protein degradation was observed according to gel electrophoresis.

In a fourth trial, water was obtained from a sewage plant prior to chlorination and poured through the Figure 1 apparatus at a rate of about 800 ml/min. The influent was found to have 1000 CFU's per ml and the effluent was found to have 1 CFU per ml. The UV transmittance of the sewage water was 60%.

The foregoing examples constituted feasibility tests, to establish the effectiveness of the present invention.

A likely application for the illustrated apparatus is in the treatment of water for human consumption, particularly, the purification of drinking water to lower pathogenic content, i.e., to lower the amount of harmful bacteria or viruses or cysts. Extensive literature exists, however, which indicates that blood and blood products can be treated by UV light. It is thus contemplated that the present invention be used in such situations where appropriate. Examples of the treatment of such liquids with light are given in United States Patent No. 5,591,457 (Bolton, January 7, 1997), United States Patent No. 5,693,049 (Mersch, December 2,1997), international patent application No. PCT/US 97/21490 (Morris, published under WO 98/22164 on May 28,1998), and United States Patent No. 5,789,150 (Margolis-Nunno *et al.,* August 4,1998). It may be the case that measures to maintain blood serum or blood products at a given temperature (or within a certain temperature range) should be taken, as described above.

If particularly hot or boiling water is treated in an apparatus of the present invention, steps should be taken, if necessary, to avoid condensation on the UV lamp(s) and reflective surfaces, so as not to diminish the intensity of UV light reaching the water in the trough.

It will be evident to a person skilled In the art given this disclosure that there are means other than those described herein for disrupting the flow of water travelling through a trough in order to achieve objects of this invention. These include, but are not limited to, roughening the surface of the trough floor and walls, elliptical protrusions, ridges, ribs, dams and barriers, both upright or vertical and transverse with respect to the general flow path of liquid. Disrupting the flow generally means to disrupt the laminar flow of the liquid. Generally, it is preferable to avoid the formation of eddies within the treatment area of the apparatus.

As mentioned above, aspects of this invention can be combined with other treatment approaches. In the treatment of drinking water, for example, a filtration system can be incorporated to operate with the present invention. Advantageously, water could be filtered after UV treatment so as to reduce the build-up or concentration of pathogens within the filter, as might occur if filtration were carried out prior to UV treatment.

## Claims

1. A process for treating an aqueous liquid, the process comprising the steps of:
passing the liquid by force of gravity through a treatment area, the treatment area comprising a series of walls, which walls are spaced and oriented to define an upwardly open trough therein, the trough defining a flow path and having an inlet end and an outlet end, for the liquid to flow under the force of gravity under ambient pressure such that the liquid flowing therethrough travels in alternatingly first and second directions, generally opposite to each other, between the inlet end and the outlet end of the trough, the liquid having an upper surface exposed to ambient pressure;
disrupting the flow of the liquid as it passes through the treatment area by means of a floor of the trough being shaped to promote uneven flow of the liquid as it passes through the trough to direct lower portions of the liquid in contact therewith toward the surface of the liquid; and
exposing the upper surface of the liquid to UV light emitted from a lamp spaced therefrom.

2. The process of claim 1, including locating the lamp such that an air space between the lamp and liquid precludes contact therebetween.

3. The process of claim 1 or 2, wherein there is no quartz layer between the lamp and surface of the liquid.

4. The process of claim 1 or 2, including the step of reflecting light emitted from the lamp onto the surface of the liquid.

5. The process of claim 1 or 2, including the step of monitoring the amount of UV light emitted from the lamp.

6. The process of claim 5, including the step of activating a warning to the user when the amount of UV light emitted from the lamp is below a predetermined amount.

7. The process of claim 1 or 2 including providing the liquid in the form of a film while passing it through the treatment area.

8. The process of claim 1 or 2 wherein the liquid has a transmittance at a wavelength of 254 nm of at least 90% and in which the UV light to which the surface is exposed is in a dosage of at least 10 milliwatt-second/cm, or wherein the liquid has a transmittance of at least 50% and in which the UV light to which the surface is exposed is in a dosage of at least 16 milliwatt-second/cm, or wherein the liquid has a transmittance of less that 50% and in which the UV light to which the surface is exposed is in a dosage of at least 25 milliwatt-second/cm or a dosage of at least 50 milliwatt-second/cm.

9. The process of claim 1 including passing the liquid by force of gravity from a liquid storage area into the treatment area.

10. The process of claim 3 including locating the lamp above the surface of the liquid.

11. The process of claim 1 wherein the lamp is a low pressure mercury vapour lamp.

12. The process of claim 11 wherein the lamp is a medium pressure mercury vapour lamp.

13. The process of claim 1 wherein the liquid is maintained at a temperature of between about 0 and 40°C preferably between about 15 and 35°C, more preferably between about 20 and 35°C while passing through the treatment area.

14. The process of claim 13 wherein the lamp is a low pressure lamp and the liquid is at a temperature in the range of from about 0 to 40°C, preferably in the range of from about 15 to 35°C, more preferably in the range of from about 20 to 35°C just prior to entering the treatment area and the liquid is passed through the treatment area at a rate suitable to maintain the temperature of the liquid within the range.

15. The process of claim 1, further comprising the step of removing particles suspended in the liquid.

16. The process of claim 15, wherein the particles include or have attached intact bacteria and/or intact viruses and/or parasitic cysts.

17. The process of claim 15 or 16 wherein the removing step is selected from processes consisting of filtration, centrifugation, flocculation, exposure to activated carbon, exposure to granulated carbon, and combinations thereof.

18. The process of claim 17 wherein the liquid is water for human consumption and the removing step includes removing organic substances that impart an undesirable smell or odour to the water.

19. The process of claim 18 wherein the removing step includes removing heavy metals from the water.

20. The process of any of claims 15 to 19 wherein the removing step is carried out after the exposing step or prior to the passing step.

21. The process of claim 1, wherein the step of disrupting the flow of the liquid includes obstructing the flow of the liquid in the treatment area.

22. The process of claim 21 wherein the liquid flows through a trough located in the treatment area and disrupting the flow of the liquid includes providing a physical barrier located in the trough.

23. The process of claim 22, wherein the liquid is passed along a path to have a major axial direction of flow and said barrier protrudes into the trough to create localized flow of the liquid in a direction non-parallel to the major axial direction.

24. The process of claim 23 wherein the barrier protrudes laterally into the trough or upwardly into the trough.

25. The process of claim 24, wherein the barrier includes a raised ridge generally transverse to the major axial direction of flow of the liquid.

26. The process of claim 25 including the step of passing the entirety of the liquid passing through the treatment over one or more of a said ridge.

27. The process of claim 21, wherein the liquid flows through a trough located in the treatment area and disrupting the flow of the liquid includes providing a depression in the trough.

28. The process of claim 7 wherein the average thickness of the film in the treatment area is up to about 5 cm, or up to about 3 cm or up to about 2 cm, preferably up to about 1cm, more preferably up to about 0.5cm, most preferably up to about 0.2 cm.

29. The process of claim 7 wherein the liquid flows through a trough at an average flow rate of up to about 500 ml per minute per cm average width of the liquid in the trough.

30. The process of claim 1 wherein the trough includes spaced apart protrusions extending into the trough, the protrusions being located along the length of the trough to mix the liquid in contact therewith.

31. The process of claim 1, wherein the liquid is blood serum or a blood product.

32. An apparatus for treating an aqueous liquid such as water with ultraviolet radiation, the apparatus comprising:
a treatment chamber comprising a series of walls, which walls are spaced and oriented to define an upwardly open trough therein, the trough defining a flow path and having an inlet end and an outlet end, for the liquid to flow under the force of gravity under ambient pressure such that the liquid flowing therethrough travels in alternatingly first and second directions, generally opposite to each other, between the inlet end and the outlet end of the trough; and
an ultraviolet lamp spaced from the flow path to preclude contact of the lamp with the liquid and located to permit exposure of a top surface of liquid in the trough to radiation emitted from the lamp; and wherein,
the trough has a floor which is shaped to promote uneven flow of the liquid as it passes through the trough to direct lower portions of the liquid in contact therewith toward the surface of the liquid.

33. The apparatus of claim 32, further comprising protrusions located in the flow path of the trough, which protrusions disrupt laminar flow and promote mixing of liquid flowing through the trough.

34. The apparatus of claim 32, wherein the apparatus is a portable counter top appliance, further comprising:
a liquid storage chamber located in an elevated location with respect to the treatment chamber, having one or more apertures in a wall thereof, the apertures being in communication with the treatment chamber to permit, under the force of gravity, controlled flow of a said liquid from the storage chamber to a said trough of the treatment chamber.

35. The apparatus of claim 34 wherein the one or more apertures are dimensioned to permit entry of up to about 3 litres per minute or up to about 2 litres per minute or up to about 1.5 litres per minute or up to about 1 litre per minute of liquid into the treatment chamber.

36. The apparatus of claim 35 wherein the trough is shaped and angled with the horizontal such that the average thickness of the liquid is no greater than about 3 cm, or no greater than about 2 cm, or no greater than about 1 cm, or no greater than about 0.5 cm.

37. The apparatus of claim 32, wherein:
the trough has an upper entry end and a lower exit end; and
the trough is shaped and angled with the horizontal, such that when a said liquid is fed to the entry end at a rate of up to about 2 litres per minute, the average thickness of the liquid flowing in the trough is no greater than about 0.3 cm.

38. The apparatus of claim 32, wherein the distance between the entry end and the exit end of the trough is sufficient to provide an average liquid residence time of at least about 3 seconds, or at least about 5 seconds or at least about 10 seconds, or at least about 15 seconds, or at least about 20 seconds.

39. The apparatus of claim 32, 33, or 34, further comprising at least one ridge located in the flow path of the trough, the ridge being shaped such that contact therewith by the flowing liquid forces lower portions of the liquid toward the surface of the liquid, wherein there are preferably two said ridges, or three said ridges, or four said ridges.

40. The apparatus of claim 32 wherein a lower end of the trough is located above an upwardly open end of a receiving chamber.

41. The apparatus of claim 40 wherein the receiving chamber is a hand-held jug.

42. The apparatus of claim 32 wherein the trough is angled between about 0° and about 15° with the horizontal, or between about 0 and about 10° with the horizontal, or between about 0 and about 5° with the horizontal, or between about 5 and about 15° with the horizontal, or 10° with the horizontal.

43. The apparatus of claim 32 wherein the lamp is a low pressure mercury lamp.

44. The apparatus of claim 43 wherein the lamp is located above the trough to permit exposure of said top surface of liquid in the trough to UV rays emitted from the lamp.

45. The apparatus of claim 32, further comprising at least one UV-reflective surface located to re-direct UV rays emitted from the lamp in a direction away from the trough toward the trough.

46. The apparatus of claim 45, wherein the lamp is in an elevated location with respect to the trough and the UV-reflective surface is located in an elevated location with respect to the trough.

47. The apparatus of claim 32 further comprising a reservoir at an elevated location with respect to the treatment chamber and in communication with the treatment chamber to permit flow of liquid from the reservoir to the inlet end of the trough of the treatment chamber.

48. The apparatus of claim 32 or 47 wherein the floor of the trough is inclined downwardly between the inlet end and outlet end to promote the flow of liquid from the inlet end toward the outlet end under the force of gravity.

49. The apparatus of claim 47 wherein the reservoir includes a housing for the liquid and the housing includes an aperture to permit said flow of liquid from the housing to the inlet end of the trough.

50. The apparatus of claim 49 wherein the housing is located above the treatment chamber and said aperture is located in a floor of the housing.

51. The apparatus of claim 50 wherein the apparatus is a portable counter top apparatus and the housing of the reservoir is dimensioned to hold up to about 3 litres of water and the aperture is dimensioned, or one or more or said apertures are together dimensioned, to provide a flow rate of up to about 2 litres per minute from the housing to the inlet end of the trough.

52. The apparatus of claim 50 wherein the treatment chamber has a roof comprising the underside of the floor of the housing.

53. The apparatus of claim 52 wherein the floor of the trough is inclined downwardly between the inlet end and outlet end to promote the flow of liquid from the inlet end toward the outlet end under the force of gravity and the floor of the housing is generally inclined downwardly toward the aperture.

54. , The apparatus of any of claims 47 to 53 wherein there is at least one protrusion extending upwardly from the floor of the trough to promote said uneven flow of the liquid as it passes through the trough, wherein there are preferably at least five said protrusions.

55. The apparatus of any of claims 47 to 54 wherein said ultraviolet lamp is located in the treatment chamber above the trough.

56. The apparatus of any of claims 47 to 55 wherein the ultraviolet lamp is a low pressure lamp and there is a reflector located to direct light emitted from the lamp in a direction away from the trough toward the trough to strike the surface of liquid in the trough.

57. The apparatus of claim 50 wherein the ultraviolet lamp is a medium pressure lamp and there is a reflector located to direct light emitted from the lamp in a direction away from the trough toward the trough to strike the surface of liquid in the trough and the apparatus further comprises means for ventilating the space between the lamp and the trough to maintain heat flow from the lamp to the liquid below a fixed amount, and / or means for cooling the liquid.

58. The apparatus of claim 32 wherein the walls are up to about 5 cm in height or up to about 3 cm in height or up to about 2 cm in height and the walls are spaced from each other such that the trough is up to about 3 cm in width or up to about 2 cm in width or up to about 1 cm in width.

## Patentansprüche

1. Verfahren zur Behandlung einer wäßrigen Flüssigkeit, wobei das Verfahren die folgenden Schritte aufweist:
Führen der Flüssigkeit durch Schwerkraft durch einen Behandlungsbereich, wobei der Behandlungsbereich eine Folge von Wänden aufweist, wobei die Wände so beabstandet und orientiert sind, daß sie einen nach oben offenen Trog darin bilden, wobei der Trog einen Durchflußweg bildet und ein Einlaßende sowie ein Auslaßende hat, damit die Flüssigkeit unter der Schwerkraft bei Umgebungsdruck so fließt, daß sich die durchfließende Flüssigkeit abwechselnd in einer ersten und einer zweiten Richtung, die allgemein zueinander entgegengesetzt sind, zwischen dem Einlaßende und dem Auslaßende des Trogs bewegt, wobei die Flüssigkeit eine Oberfläche hat, die Umgebungsdruck ausgesetzt ist;
Stören des Flusses der Flüssigkeit bei ihrem Durchlauf durch den Behandlungsbereich mittels eines Bodens des Trogs, der so geformt ist, daß er ungleichmäßigen Fluß der Flüssigkeit bei ihrem Durchlauf durch den Trog fördert, um untere Abschnitte der Flüssigkeit in Kontakt mit ihm zur Oberfläche der Flüssigkeit zu leiten; und
Einwirkenlassen von UV-Licht auf die Oberfläche der Flüssigkeit, das von einer von ihr beabstandeten Lampe abgestrahlt wird.

2. Verfahren nach Anspruch 1 mit dem Schritt des Anordnens der Lampe derart, daß ein Luftraum zwischen der Lampe und der Flüssigkeit Kontakt zwischen ihnen verhindert.

3. Verfahren nach Anspruch 1 oder 2, wobei keine Quarzschicht zwischen der Lampe und der Oberfläche der Flüssigkeit vorhanden ist.

4. Verfahren nach Anspruch 1 oder 2 mit dem Schritt des Reflektierens von Licht, das von der Lampe auf die Oberfläche der Flüssigkeit abgestrahlt wird.

5. Verfahren nach Anspruch 1 oder 2 mit dem Schritt des Überwachens der Menge von UV-Licht, das von der Lampe abgestrahlt wird.

6. Verfahren nach Anspruch 5 mit dem Schritt des Aktivierens einer Warnung für den Benutzer, wenn die Menge von UV-Licht, das von der Lampe abgestrahlt wird, unter einer vorbestimmten Menge liegt.

7. Verfahren nach Anspruch 1 oder 2 mit dem Schritt des Bereitstellens der Flüssigkeit in Form eines Films, während sie durch den Behandlungsbereich geführt wird.

8. Verfahren nach Anspruch 1 oder 2, wobei die Flüssigkeit eine Durchlässigkeit bei einer Wellenlänge von 254 nm von mindestens 90 % hat und das UV-Licht, dem die Oberfläche ausgesetzt ist, eine Dosierung von mindestens 10 Milliwattsekunden/cm hat, oder wobei die Flüssigkeit eine Durchlässigkeit von mindestens 50 % hat und das UV-Licht, dem die Oberfläche ausgesetzt ist, eine Dosierung von mindestens 16 Milliwattsekunden/cm hat, oder wobei die Flüssigkeit eine Durchlässigkeit von weniger als 50 % hat und das UV-Licht, dem die Oberfläche ausgesetzt ist, eine Dosierung von mindestens 25 Milliwattsekunden/cm oder eine Dosierung von mindestens 50 Milliwattsekunden/cm hat.

9. Verfahren nach Anspruch 1 mit dem Schritt des Führens der Flüssigkeit durch Schwerkraft aus einem Flüssigkeitsspeicherbereich in den Behandlungsbereich.

10. Verfahren nach Anspruch 3 mit dem Schritt des Anordnens der Lampe über der Oberfläche der Flüssigkeit.

11. Verfahren nach Anspruch 1, wobei die Lampe eine Niederdruck-Quecksilberdampflampe ist.

12. Verfahren nach Anspruch 11, wobei die Lampe eine Mitteldruck-Quecksilberdampflampe ist.

13. Verfahren nach Anspruch 1, wobei die Flüssigkeit auf einer Temperatur zwischen etwa 0 und 40 °C, vorzugsweise zwischen etwa 15 und 35 °C, besonders bevorzugt zwischen etwa 20 und 35 °C gehalten wird, während sie durch den Behandlungsbereich geführt wird.

14. Verfahren nach Anspruch 13, wobei die Lampe eine Niederdrucklampe ist und die Flüssigkeit kurz vor Eintritt in den Behandlungsbereich eine Temperatur im Bereich von etwa 0 bis 40 °C, vorzugsweise zwischen etwa 15 und 35 °C, besonders bevorzugt zwischen etwa 20 und 35 °C hat und die Flüssigkeit durch den Behandlungsbereich mit einer geeigneten Geschwindigkeit geführt wird, um die Temperatur der Flüssigkeit in dem Bereich zu halten.

15. Verfahren nach Anspruch 1, ferner mit dem Schritt des Entfernens von Teilchen, die in der Flüssigkeit suspendiert sind.

16. Verfahren nach Anspruch 15, wobei die Teilchen intakte Bakterien und/oder intakte Viren und/oder Parasitenzysten aufweisen oder diese daran angelagert sind.

17. Verfahren nach Anspruch 15 oder 16, wobei der Entfernungsschritt aus Verfahren ausgewählt ist, die aus Filtern, Zentrifugieren, Ausflocken, Einwirkenlassen von Aktivkohle, Einwirkenlassen von Kornkohle und deren Kombinationen bestehen.

18. Verfahren nach Anspruch 17, wobei die Flüssigkeit Wasser zum menschlichen Verbrauch ist und der Entfernungsschritt den Schritt des Entfernens organischer Stoffe aufweist, die dem Wasser einen unerwünschten Geschmack oder Geruch geben.

19. Verfahren nach Anspruch 18, wobei der Schritt des Entfernens den Schritt des Entfernens von Schwermetallen aus dem Wasser aufweist.

20. Verfahren nach einem der Ansprüche 15 bis 19, wobei der Entfernungsschritt nach dem Schritt des Einwirkenlassens oder vor dem Durchführschritt durchgeführt wird.

21. Verfahren nach Anspruch 1, wobei der Schritt des Störens des Flusses der Flüssigkeit den Schritt des Behinderns des Flusses der Flüssigkeit im Behandlungsbereich aufweist.

22. Verfahren nach Anspruch 21, wobei die Flüssigkeit einen Trog durchfließt, der im Behandlungsbereich angeordnet ist, und der Schritt des Störens des Flusses der Flüssigkeit den Schritt des Bereitstellens einer körperlichen Sperre aufweist, die im Trog angeordnet ist.

23. Verfahren nach Anspruch 22, wobei die Flüssigkeit so auf einem Weg geführt wird, daß sie eine axiale Hauptflußrichtung hat, und die Sperre in den Trog vorsteht, um lokalisierten Fluß der Flüssigkeit in einer nicht parallelen Richtung zur axialen Hauptrichtung zu erzeugen.

24. Verfahren nach Anspruch 23, wobei die Sperre seitlich oder nach oben in den Trog vorsteht.

25. Verfahren nach Anspruch 24, wobei die Sperre einen erhöhten Steg aufweist, der allgemein quer zur axialen Hauptflußrichtung der Flüssigkeit ist.

26. Verfahren nach Anspruch 25 mit dem Schritt des Führens der Gesamtheit der Flüssigkeit, die die Behandlung durchläuft, über einen oder mehrere der Stege.

27. Verfahren nach Anspruch 21, wobei die Flüssigkeit einen Trog durchfließt, der im Behandlungsbereich angeordnet ist, und der Schritt des Störens des Flusses der Flüssigkeit den Schritt des Bereitstellens einer Vertiefung im Trog aufweist.

28. Verfahren nach Anspruch 7, wobei die mittlere Stärke des Films im Behandlungsbereich bis etwa 5 cm oder bis etwa 3 cm oder bis etwa 2 cm, vorzugsweise bis etwa 1 cm, bevorzugt bis etwa 0,5 cm, besonders bevorzugt bis etwa 0,2 cm beträgt.

29. Verfahren nach Anspruch 7, wobei die Flüssigkeit einen Trog mit einer mittleren Flußgeschwindigkeit bis etwa 600 ml pro Minute pro cm mittlerer Breite der Flüssigkeit im Trog durchfließt.

30. Verfahren nach Anspruch 1, wobei der Trog beabstandete Vorwölbungen aufweist, die sich in den Trog erstrecken, wobei die Vorwölbungen über die Länge des Trogs so angeordnet sind, daß sie die mit ihnen in Kontakt stehende Flüssigkeit mischen.

31. Verfahren nach Anspruch 1, wobei die Flüssigkeit Blutserum oder ein Blutprodukt ist.

32. Vorrichtung zur Behandlung einer wäßrigen Flüssigkeit, z. B. Wasser, mit Ultraviolettstrahlung, wobei die Vorrichtung aufweist:
eine Behandlungskammer mit einer Folge von Wänden, wobei die Wände so beabstandet und orientiert sind, daß sie einen nach oben offenen Trog darin bilden, wobei der Trog einen Durchflußweg bildet und ein Einlaßende sowie ein Auslaßende hat, damit die Flüssigkeit unter sowie ein Auslaßende hat, damit die Flüssigkeit unter der Schwerkraft bei Umgebungsdruck so fließt, daß sich die durchfließende Flüssigkeit abwechselnd in einer ersten und einer zweiten Richtung, die allgemein zueinander entgegengesetzt sind, zwischen dem Einlaßende und
dem Auslaßende des Trogs bewegt; und
eine Ultraviolettlampe, die vom Durchflußweg so beabstandet ist, daß Kontakt der Lampe mit der Flüssigkeit verhindert ist, und die so angeordnet ist, daß von der Lampe abgestrahlte Strahlung auf eine Oberfläche der Flüssigkeit im Trog einwirken kann; und wobei
der Trog einen Boden hat, der so geformt ist, daß er ungleichmäßigen Fluß der Flüssigkeit bei ihrem Durchlauf durch den Trog fördert, um untere Abschnitte der Flüssigkeit in Kontakt mit ihm zur Oberfläche der Flüssigkeit zu leiten.

33. Vorrichtung nach Anspruch 32, ferner mit Vorwölbungen, die im Durchflußweg des Trogs angeordnet sind, wobei die Vorwölbungen laminare Strömung stören und das Mischen von Flüssigkeit fördern, die den Trog durchfließt.

34. Vorrichtung nach Anspruch 32, wobei die Vorrichtung ein tragbares Tischgerät ist, das ferner aufweist:
eine Flüssigkeitsspeicherkammer, die an einer erhöhten Stelle im Hinblick auf die Behandlungskammer angeordnet ist, mit einer oder mehreren Öffnungen in einer Wand von ihr, wobei die Öffnungen mit der Behandlungskammer kommunizieren, um unter der Schwerkraft einen gesteuerten Durchfluß der Flüssigkeit aus der Speicherkammer zum Trog der Behandlungskammer zu ermöglichen.

35. Vorrichtung nach Anspruch 34, wobei die eine oder die mehreren Öffnungen so bemessen sind, daß sie den Eintritt von Flüssigkeit in die Behandlungskammer mit bis etwa 3 Litern pro Minute oder bis etwa 2 Litern pro Minute oder bis etwa 1,5 Litern pro Minute oder bis etwa 1 Liter pro Minute ermöglichen.

36. Vorrichtung nach Anspruch 35, wobei der Trog so geformt und zur Horizontalen abgewinkelt ist, daß die mittlere Stärke der Flüssigkeit höchstens etwa 3 cm, oder höchstens etwa 2 cm, oder höchstens etwa 1 cm, oder höchstens etwa 0,5 cm beträgt.

37. Vorrichtung nach Anspruch 32, wobei:
der Trog ein oberes Eingangsende und ein unteres Austrittsende hat; und
der Trog so geformt und zur Horizontalen abgewinkelt ist, daß bei Zufuhr der Flüssigkeit zum Eingangsende mit einer Rate bis etwa 2 Litern pro Minute die mittlere Stärke der im Trog fließenden Flüssigkeit höchstens etwa 0,3 cm beträgt.

38. Vorrichtung nach Anspruch 32, wobei der Abstand zwischen dem Eingangsende und dem Austrittsende des Trogs ausreicht, für eine mittlere Flüssigkeitsverweilzeit von mindestens etwa 3 Sekunden, oder mindestens etwa 5 Sekunden, oder mindestens etwa 10 Sekunden, oder mindestens etwa 15 Sekunden, oder mindestens etwa 20 Sekunden zu sorgen.

39. Vorrichtung nach Anspruch 32, 33 oder 34, ferner mit mindestens einem Steg vorzugsweise zwei oder drei oder vier Stegen, der bzw. die im Flußweg des Trogs angeordnet ist (sind), wobei der mindestens eine Steg so geformt ist, daß ein Kontakt der fließenden Flüssigkeit mit ihm untere Abschnitte der Flüssigkeit zur Oberfläche der Flüssigkeit drückt.

40. Vorrichtung nach Anspruch 32, wobei ein unteres Ende des Trogs über einem nach oben offenen Ende einer Aufnahmekammer angeordnet ist.

41. Vorrichtung nach Anspruch 40, wobei die Aufnahmekammer ein Handkrug ist.

42. Vorrichtung nach Anspruch 32, wobei der Trog zwischen etwa 0° und etwa 15°, oder zwischen etwa 0 und etwa 10°, oder zwischen etwa 0 und etwa 5°, oder zwischen etwa 5 und etwa 15°, öder 10° zur Horizontalen abgewinkelt ist.

43. Vorrichtung nach Anspruch 32, wobei die Lampe eine Niederdruck-Quecksilberlampe ist.

44. Vorrichtung nach Anspruch 43, wobei die Lampe über dem Trog angeordnet ist, damit von der Lampe abgestrahlte UV-Strahlen auf die Oberfläche der Flüssigkeit im Trog einwirken können.

45. Vorrichtung nach Anspruch 32, ferner mit mindestens einer UV-reflektierenden Oberfläche, die so angeordnet ist, daß sie von der Lampe in einer vom Trog wegführenden Richtung abgestrahlte UV-Strahlen wieder zum Trog leitet.

46. Vorrichtung nach Anspruch 45, wobei sich die Lampe an einer erhöhten Stelle im Hinblick auf den Trog befindet und die UV-reflektierende Oberfläche an einer erhöhten Stelle im Hinblick auf den Trog angeordnet ist.

47. Vorrichtung nach Anspruch 32, ferner mit einem Reservoir an einer erhöhten Stelle im Hinblick auf die Behandlungskammer, das mit der Behandlungskammer kommuniziert, um den Fluß von Flüssigkeit aus dem Reservoir zum Einlaßende des Trogs der Behandlungskammer zu ermöglichen.

48. Vorrichtung nach Anspruch 32 oder 47, wobei der Boden des Trogs zwischen dem Einlaßende und Auslaßende nach unten geneigt ist, um den Durchfluß von Flüssigkeit vom Einlaßende zum Auslaßende unter der Schwerkraft zu fördern.

49. Vorrichtung nach Anspruch 47, wobei das Reservoir ein Gehäuse für die Flüssigkeit aufweist und das Gehäuse eine Öffnung aufweist, um den Fluß von Flüssigkeit aus dem Gehäuse zum Einlaßende des Trogs zu ermöglichen.

50. Vorrichtung nach Anspruch 49, wobei das Gehäuse über der Behandlungskammer angeordnet ist und die Öffnung in einem Boden des Gehäuses angeordnet ist.

51. Vorrichtung nach Anspruch 50, wobei die Vorrichtung eine tragbare Tischvorrichtung ist und das Gehäuse des Reservoirs so bemessen ist, daß es etwa 3 Liter Wasser enthält, und die Öffnung so bemessen ist oder eine oder mehrere Öffnungen zusammen so bemessen sind, daß sie für eine Flußgeschwindigkeit bis etwa 2 Litern pro Minute vom Gehäuse zum Einlaßende des Trogs sorgen.

52. Vorrichtung nach Anspruch 50, wobei die Behandlungskammer ein Dach hat, das die Unterseite des Bodens des Gehäuses aufweist.

53. Vorrichtung nach Anspruch 52, wobei der Boden des Trogs zwischen dem Einlaßende und Auslaßende nach unten geneigt ist, um den Fluß von Flüssigkeit vom Einlaßende zum Auslaßende unter der Schwerkraft zu fördern, und der Boden des Gehäuses allgemein nach unten zur Öffnung geneigt ist.

54. Vorrichtung nach einem der Ansprüche 47 bis 53, wobei mindestens eine Vorwölbung vorzugsweise mindestens fünf Vorwölbungen vorhanden ist (sind), die sich vom Boden des Trogs nach oben erstrecken, um den ungleichmäßigen Fluß der Flüssigkeit bei ihrem Durchlauf durch den Trog zu fördern.

55. Vorrichtung nach einem der Ansprüche 47 bis 54, wobei die Ultraviolettlampe in der Behandlungskammer über dem Trog angeordnet ist.

56. Vorrichtung nach einem der Ansprüche 47 bis 55, wobei die Ultraviolettlampe eine Niederdrucklampe ist und ein Reflektor vorhanden ist, der so angeordnet ist, daß er von der Lampe in einer vom Trog wegführenden Richtung abgestrahltes Licht zum Trog leitet, damit es auf die Oberfläche der Flüssigkeit im Trog trifft.

57. Vorrichtung nach Anspruch 50, wobei die Ultraviolettlampe eine Mitteldrucklampe ist und ein Reflektor vorhanden ist, der so angeordnet ist, daß er von der Lampe in einer vom Trog wegführenden Richtung abgestrahltes Licht zum Trog leitet, damit es auf die Oberfläche der Flüssigkeit im Trog trifft, und die Vorrichtung ferner eine Einrichtung zum Kühlen der Flüssigkeit und/oder eine Einrichtung zum Belüften des Raums zwischen der Lampe und dem Trog aufweist, um Wärmefluß von der Lampe zur Flüssigkeit unter einer festgelegten Menge zu halten.

58. Vorrichtung nach Anspruch 32, wobei die Wände bis etwa 5 cm hoch oder bis etwa 3 cm hoch oder bis etwa 2 cm hoch sind und die Wände voneinander so beabstandet sind, daß der Trog bis etwa 3 cm breit oder bis etwa 2 cm breit oder bis etwa 1 cm breit ist.

## Revendications

1. Procédé pour traiter un liquide aqueux, procédé comprenant les étapes consistant à :
faire passer le liquide sous l'effet de la force de gravité à travers une zone de traitement, la zone de traitement comprenant une série de parois, lesquelles parois sont espacées et orientées pour y définir une goulotte ouverte vers le haut, la goulotte définissant un trajet d'écoulement et ayant une extrémité d'entrée et une extrémité de sortie, pour que le liquide s'écoule sous l'effet de la force de gravité à la pression ambiante de façon à ce que le liquide s'y écoulant se déplace, alternativement, dans des première et seconde directions, généralement opposées l'une à l'autre, entre l'extrémité d'entrée et l'extrémité de sortie de la goulotte, le liquide ayant une surface supérieure exposée à la pression ambiante ;
perturber l'écoulement du liquide lors de son passage dans la zone de traitement au moyen d'un plancher de la goulotte façonné pour favoriser un écoulement irrégulier du liquide lors de son passage dans la goulotte afin de diriger les parties inférieures du liquide en contact avec celui-ci vers la surface du liquide ; et
exposer la surface supérieure du liquide à une lumière ultraviolette émise par une lampe espacée de celle-ci.

2. Procédé selon la revendication 1, comprenant le positionnement de la lampe de façon à ce qu'un espace vide entre la lampe et le liquide empêche un contact entre eux.

3. Procédé selon la revendication 1 ou 2, dans lequel on ne trouve aucune couche de quartz entre la lampe et la surface du liquide.

4. Procédé selon la revendication 1 ou 2, comprenant l'étape consistant à réfléchir la lumière émise par la lampe sur la surface du liquide.

5. Procédé selon la revendication 1 ou 2, comprenant l'étape consistant à surveiller la quantité de lumière ultraviolette émise par la lampe.

6. Procédé selon la revendication 5, comprenant l'étape consistant à activer un dispositif d'avertissement de l'utilisateur lorsque la quantité de lumière ultraviolette émise par la lampe est inférieure à une quantité prédéterminée.

7. Procédé selon la revendication 1 ou 2, comprenant la fourniture du liquide sous forme de film lors de son passage dans la zone de traitement.

8. Procédé selon la revendication 1 ou 2, dans lequel le liquide a un facteur de transmission à une longueur d'onde de 254 nm d'au moins 90% et la lumière ultraviolette à laquelle la surface est exposée est à un dosage d'au moins 10 milliwatts-seconde/cm ou dans lequel le liquide a un facteur de transmission d'au moins 50% et la lumière ultraviolette à laquelle la surface est exposée est à un dosage d'au moins 16 milliwatts-seconde/cm ou dans lequel le liquide a un facteur de transmission inférieur à 50% et la lumière ultraviolette à laquelle la surface est exposée est à un dosage d'au moins 25 milliwatts-seconde/cm ou un dosage d'au moins 50 milliwatts-seconde/cm.

9. Procédé selon la revendication 1, comprenant le passage du liquide sous l'effet de la force de gravité d'une zone de stockage du liquide à la zone de traitement.

10. Procédé selon la revendication 3, comprenant le positionnement de la lampe au-dessus de la surface du liquide.

11. Procédé selon la revendication 1, dans lequel la lampe est une lampe à vapeur de mercure à basse pression.

12. Procédé selon la revendication 11, dans lequel la lampe est une lampe à vapeur de mercure à moyenne pression.

13. Procédé selon la revendication 1, dans lequel le liquide est maintenu à une température comprise entre environ 0 et 40°C, de préférence entre environ 15 et 35 °C, particulièrement préféré entre environ 20 et 35 °C, lors de son passage dans la zone de traitement.

14. Procédé selon la revendication 13, dans lequel la lampe est une lampe à basse pression et le liquide est à une température dans la plage d'environ 0 à 40°C, de préférence dans la plage d'environ 15 à 35 °C, particulièrement préféré dans la plage d'environ 20 à 35 °C, juste avant de pénétrer dans la zone de traitement et le liquide traverse la zone de traitement à une vitesse appropriée pour maintenir la température du liquide dans cette plage.

15. Procédé selon la revendication 1, comprenant en outre l'étape consistant à extraire des particules en suspension dans le liquide.

16. Procédé selon la revendication 15, dans lequel les particules comprennent ou sont liées à des bactéries intactes et/ou des virus intacts et/ou des kystes parasitaires.

17. Procédé selon la revendication 15 ou 16, dans lequel l'étape d'extraction est choisie parmi les procédés constitués par la filtration, la centrifugation, la floculation, l'exposition au charbon actif, l'exposition au charbon granulé, et leurs combinaisons.

18. Procédé selon la revendication 17, dans lequel le liquide est de l'eau destinée à la consommation humaine et l'étape d'extraction comprend l'extraction des substances organiques qui donnent une odeur indésirable à l'eau.

19. Procédé selon la revendication 18, dans lequel l'étape d'extraction comprend l'extraction des métaux lourds de l'eau.

20. Procédé selon l'une quelconque des revendications 15 à 19, dans lequel l'étape d'extraction est effectuée après l'étape d'exposition ou avant l'étape de passage.

21. Procédé selon la revendication 1, dans lequel l'étape consistant à perturber l'écoulement du liquide comprend l'obstruction de l'écoulement du liquide dans la zone de traitement.

22. Procédé selon la revendication 21, dans lequel le liquide s'écoule à travers une goulotte positionnée dans la zone de traitement et la perturbation de l'écoulement du liquide comprend la mise en place d'une barrière physique positionnée dans la goulotte.

23. Procédé selon la revendication 22, dans lequel on fait passer le liquide le long d'un trajet pour avoir une direction axiale principale d'écoulement et ladite barrière fait saillie dans la goulotte pour créer un écoulement localisé du liquide dans une direction non parallèle à la direction axiale principale.

24. Procédé selon la revendication 23, dans lequel la barrière fait saillie latéralement ou vers le haut dans la goulotte.

25. Procédé selon la revendication 24, dans lequel la barrière comprend une arête surélevée généralement transversale à la direction axiale principale d'écoulement du liquide.

26. Procédé selon la revendication 25, comprenant l'étape consistant à faire passer la totalité du liquide subissant le traitement sur une ou plusieurs d'une dite arête.

27. Procédé selon la revendication 21, dans lequel le liquide s'écoule à travers une goulotte positionnée dans la zone de traitement et la perturbation de l'écoulement du liquide comprend la présence d'une dépression dans la goulotte.

28. Procédé selon la revendication 7, dans lequel l'épaisseur moyenne du film dans la zone de traitement va jusqu'à environ 5 cm, ou jusqu'à environ 3 cm ou jusqu'à environ 2 cm, de préférence jusqu'à environ 1 cm, de préférence jusqu'à environ 0,5 cm, particulièrement préféré jusqu'à environ 0,2 cm.

29. Procédé selon la revendication 7, dans lequel le liquide s'écoule à travers une goulotte à un débit moyen pouvant aller jusqu'à environ 500 ml par minute par cm de largeur moyenne du liquide dans la goulotte.

30. Procédé selon la revendication 1, dans lequel la goulotte comprend des protubérances espacées s'étendant dans la goulotte, les protubérances étant positionnées le long de la goulotte pour mélanger le liquide en contact avec celles-ci.

31. Procédé selon la revendication 1, dans lequel le liquide est du sérum sanguin ou un produit sanguin.

32. Appareil pour traiter un liquide aqueux tel que l'eau avec un rayonnement ultraviolet, l'appareil comprenant :
une chambre de traitement comprenant une série de parois, lesquelles parois sont espacées et orientées pour y définir une goulotte ouverte vers le haut, la goulotte définissant un trajet d'écoulement et ayant une extrémité d'entrée et une extrémité de sortie, pour que le liquide s'écoule sous l'effet de la force de gravité à la pression ambiante de façon à ce que le liquide s'y écoulant se déplace, alternativement, dans des première et seconde directions, généralement opposées l'une à l'autre, entre l'extrémité d'entrée et l'extrémité de sortie de la goulotte ; et
une lampe à ultraviolets espacée du trajet d'écoulement pour empêcher un contact de la lampe avec le liquide et positionnée pour permettre l'exposition d'une surface supérieure de liquide dans la goulotte au rayonnement émis par la lampe ; et dans lequel,
la goulotte a un plancher qui est façonné pour favoriser un écoulement irrégulier du liquide lors de son passage dans la goulotte afin de diriger les parties inférieures du liquide en contact avec celui-ci vers la surface du liquide.

33. Appareil selon la revendication 32, comprenant en outre des protubérances positionnées dans le trajet d'écoulement de la goulotte, lesquelles protubérances perturbent l'écoulement laminaire et favorisent le mélange du liquide s'écoulant à travers la goulotte.

34. Appareil selon la revendication 32, lequel appareil est un dispositif portable pour plan de travail, comprenant en outre :
une chambre de stockage de liquide positionnée à un emplacement élevé par rapport à la chambre de traitement, ayant une ou plusieurs ouvertures dans une de ces parois, les ouvertures étant en communication avec la chambre de traitement pour permettre, sous l'effet de la force de gravité, un écoulement contrôlé d'un dit liquide de la chambre de stockage dans une dite goulotte de la chambre de traitement.

35. Appareil selon la revendication 34, dans lequel les une ou plusieurs ouvertures sont dimensionnées pour permettre une entrée de liquide dans la chambre de traitement allant jusqu'à environ 3 litres par minute ou jusqu'à environ 2 litres par minute ou jusqu'à environ 1,5 litres par minute ou jusqu'à environ 1 litre par minute.

36. Appareil selon la revendication 35, dans lequel la goulotte est façonnée et fait un angle avec l'horizontale de façon à ce que l'épaisseur moyenne du liquide ne soit pas supérieure à environ 3 cm ou ne soit pas supérieure à environ 2 cm ou ne soit pas supérieure à environ 1 cm ou ne soit pas supérieure à environ 0,5 cm.

37. Appareil selon la revendication 32, dans lequel :
la goulotte a une extrémité d'entrée supérieure et une extrémité de sortie inférieure ; et
la goulotte est façonnée et fait un angle avec l'horizontale, de façon à ce que lorsqu'un dit liquide est chargé dans l'extrémité d'entrée à un taux allant jusqu'à environ 2 litres par minute, l'épaisseur moyenne du liquide s'écoulant dans la goulotte ne soit pas supérieure à environ 0,3 cm.

38. Appareil selon la revendication 32, dans lequel la distance entre l'extrémité d'entrée et l'extrémité de sortie de la goulotte est suffisante pour obtenir un temps de séjour moyen du liquide d'au moins environ 3 secondes ou d'au moins environ 5 secondes ou d'au moins environ 10 secondes ou d'au moins environ 15 secondes ou d'au moins environ 20 secondes.

39. Appareil selon la revendication 32, 33 ou 34, comprenant en outre au moins une arête, de préférence deux ou trois ou quatre arêtes, positionnée(s) dans le trajet d'écoulement de la goulotte, l'au moins un arête étant façonnée de façon à ce que le contact avec celle-ci du liquide s'écoulant entraîne les parties inférieures du liquide vers la surface du liquide.

40. Appareil selon la revendication 32, dans lequel une extrémité inférieure de la goulotte est positionnée au-dessus d'une extrémité ouverte vers le haut d'une chambre réceptrice.

41. Appareil selon la revendication 40, dans lequel la chambre réceptrice est une carafe à anse.

42. Appareil selon la revendication 32, dans lequel la goulotte fait un angle compris entre environ 0° et environ 15° ou entre environ 0 et environ 10° ou entre environ 0 et environ 5° ou entre environ 5 et environ 15° ou un angle de 10° avec l'horizontale.

43. Appareil selon la revendication 32, dans lequel la lampe est une lampe à mercure à basse pression.

44. Appareil selon la revendication 43, dans lequel la lampe est positionnée au-dessus de la goulotte pour permettre l'exposition de ladite surface supérieure de liquide dans la goulotte aux rayons ultraviolets émis par la lampe.

45. Appareil selon la revendication 32, comprenant en outre au moins une surface réfléchissant les ultraviolets positionnée pour rediriger les rayons ultraviolets émis par la lampe dans une direction éloignée de la goulotte vers la goulotte.

46. Appareil selon la revendication 45, dans lequel la lampe est à un emplacement élevé par rapport à la goulotte et la surface réfléchissant les ultraviolets est positionnée à un emplacement élevé par rapport à la goulotte.

47. Appareil selon la revendication 32, comprenant en outre un réservoir à un emplacement élevé par rapport à la chambre de traitement et en communication avec la chambre de traitement pour permettre un écoulement de liquide du réservoir vers l'extrémité d'entrée de la goulotte de la chambre de traitement.

48. Appareil selon la revendication 32 ou 47, dans lequel le plancher de la goulotte est incliné vers le bas entre l'extrémité d'entrée et l'extrémité de sortie pour favoriser l'écoulement de liquide de l'extrémité d'entrée vers l'extrémité de sortie sous l'effet de la force de gravité.

49. Appareil selon la revendication 47, dans lequel le réservoir comprend un logement pour le liquide et le logement comprend une ouverture pour permettre ledit écoulement de liquide du logement vers l'extrémité d'entrée de la goulotte.

50. Appareil selon la revendication 49, dans lequel le logement est positionné au-dessus de la chambre de traitement et ladite ouverture est positionnée dans un plancher du logement.

51. Appareil selon la revendication 50, lequel appareil est un appareil portable pour plan de travail et le logement du réservoir est dimensionné pour contenir jusqu'à environ 3 litres d'eau et l'ouverture est dimensionnée, ou une ou plusieurs desdites ouvertures sont ensemble dimensionnées pour fournir un débit allant jusqu'à environ 2 litres par minute du logement vers l'extrémité d'entrée de la goulotte.

52. Appareil selon la revendication 50, dans lequel la chambre de traitement a un toit comprenant la sous-face du plancher du logement.

53. Appareil selon la revendication 52, dans lequel le plancher de la goulotte est incliné vers le bas entre l'extrémité d'entrée et l'extrémité de sortie pour favoriser l'écoulement de liquide de l'extrémité d'entrée vers l'extrémité de sortie sous l'effet de la force de gravité et le plancher du logement est généralement incliné vers le bas vers l'ouverture.

54. Appareil selon l'une quelconque des revendications 47 à 53, dans lequel on trouve au moins une protubérance, de préférence au moins cinq desdites protubérances, s'étendant vers le haut depuis le plancher de la goulotte pour favoriser ledit écoulement irrégulier du liquide lors de son passage à travers la goulotte.

55. Appareil selon l'une quelconque des revendications 47 à 54, dans lequel ladite lampe à ultraviolets est positionnée dans la chambre de traitement au-dessus de la goulotte.

56. Appareil selon l'une quelconque des revendications 47 à 55, dans lequel la lampe à ultraviolets est une lampe à basse pression et on trouve un réflecteur positionné pour diriger la lumière émise par la lampe dans une direction éloignée de la goulotte vers la goulotte pour frapper la surface du liquide dans la goulotte.

57. Appareil selon la revendication 50, dans lequel la lampe à ultraviolets est une lampe à moyenne pression et on trouve un réflecteur positionné pour diriger la lumière émise par la lampe dans une direction éloignée de la goulotte vers la goulotte pour frapper la surface du liquide dans la goulotte et l'appareil comprend en outre un moyen pour refroidir le liquide et/ou un moyen pour ventiler l'espace entre la lampe et la goulotte afin de maintenir le flux de chaleur allant de la lampe au liquide en-dessous d'une quantité fixée.

58. Appareil selon la revendication 32, dans lequel les parois font jusqu'à environ 5 cm de haut ou jusqu'à environ 3 cm de haut ou jusqu'à environ 2 cm de haut et les parois sont espacées les unes des autres de sorte que la goulotte fait jusqu'à environ 3 cm de large ou jusqu'à environ 2 cm de large ou jusqu'à environ 1 cm de large.
